(19) Europäisches Patentamt
European Patent Office
Office européen des brevets

(11) EP 2 370 103 B1

(12) EUROPEAN PATENT SPECIFICATION

(45) Date of publication and mention of the grant of the patent:
**23.03.2016 Bulletin 2016/12**

(21) Application number: **09756754.9**

(22) Date of filing: **25.11.2009**

(51) Int Cl.:
***A61K 31/395*** *(2006.01)* ***A61K 31/5377*** *(2006.01)*
***A61K 39/395*** *(2006.01)*

(86) International application number:
**PCT/EP2009/065861**

(87) International publication number:
**WO 2010/060939 (03.06.2010 Gazette 2010/22)**

(54) **PHARMACEUTICAL COMBINATIONS COMPRISING AN ISOXAZOLE DERIVED HSP90-INHIBITOR AND THE HER2 INHIBITOR TRASTUZUMAB**

ARZNEIMITTELKOMBINATIONEN, ENTHALTEND EINEN VON ISOXAZOL ABGELEITETEN HSP90-HEMMER UND DEN HER2-HEMMER TRASTUZUMAB

COMBINAISONS PHARMACEUTIQUES RENFERMANT UN INHIBITEUR DE HSP90 DÉRIVÉ DE L'ISOXAZOLE ET L'INHIBITEUR DE HER2 TRASTUZUMAB

(84) Designated Contracting States:
**AT BE BG CH CY CZ DE DK EE ES FI FR GB GR HR HU IE IS IT LI LT LU LV MC MK MT NL NO PL PT RO SE SI SK SM TR**
Designated Extension States:
**AL BA RS**

(30) Priority: **28.11.2008 EP 08170255**
**28.11.2008 EP 08170261**

(43) Date of publication of application:
**05.10.2011 Bulletin 2011/40**

(73) Proprietor: **Novartis AG**
**4056 Basel (CH)**

(72) Inventors:
- **GARCIA-ECHEVERRIA, Carlos**
  **CH-4002 Basel (CH)**
- **JENSEN, Michael**
  **CH-4002 Basel (CH)**

(74) Representative: **Roth, Peter Richard et al**
**Novartis Pharma AG**
**Patent Department**
**4002 Basel (CH)**

(56) References cited:
**WO-A-2007/041362** **WO-A1-2004/072051**
**WO-A2-2007/053284**

- **MODI S ET AL: "Tanespimycin (an Hsp90 inhibitor) and trastuzumab is an active combination in patients (pts) with Her2-positive trastuzumab-refractory metastatic breast cancer (MBC): phase 2 trial" BREAST CANCER RESEARCH AND TREATMENT, 30TH ANNUAL SAN ANTONIO BREAST CANCER SYMPOSIUM; SAN ANTONIO, TX, USA; DECEMBER 13 -16, 2007, vol. 106, no. Suppl. 1, 1 December 2007 (2007-12-01), pages S269-S270, XP002521367**
- **LEOW CHING CHING ET AL: "IPI-504, a selective Hsp90 inhibitor is biologically active and tolerated in combination with trastuzumab in preclinical models of HER2 positive tumor xenografts" PROCEEDINGS OF THE ANNUAL MEETING OF THE AMERICAN ASSOCIATION FOR CANCER RESEARCH, NEW YORK, NY, vol. 49, 1 April 2008 (2008-04-01), pages 952-953, XP002521429 ISSN: 0197-016X**
- **MODI S; STOPECK A; GORDON M S; SOLIT D; BAGATELLE R; FLORES S; COHEN J ET AL: "Trastuzumab (T) and KOS-953 (17-AAG) is feasible and active in patients (pts) with metastatic breast cancer: preliminary results of a phase 1/2 study." BREAST CANCER RESEARCH AND TREATMENT, vol. 94, no. Suppl. 1, 2005, page S72, XP002595758 28TH ANNUAL SAN ANTONIO BREAST CANCER SYMPOSIUM; SAN ANTONIO, TX, USA; DECEMBER 08 -11, 2005 ISSN: 0167-6806**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 370 103 B1

## Description

This application claims priority to European Application Numbers 08170255.7, filed November 28, 2008, and 08170261.5, filed November 28, 2008.

Field of the Invention

The present disclosure is directed to a pharmaceutical composition comprising an Hsp90 inhibitor and one or more pharmaceutically active agent, e.g. a HER2 inhibitor, and the uses of such a composition for the treatment of disease, including proliferative diseases.

Related Background Art

In spite of numerous treatment options for proliferative disease patients, there remains a need for effective and safe antiproliferative agents and a need for their use in combination therapy.

Heat shock protein 90 (Hsp90) is recognized as an anti-cancer target. Hsp90 is a ubiquitous, highly abundant (1-2% of the total cellular protein), essential protein which functions as a molecular chaperone to ensure the conformational stability, shape and function of client proteins.

Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003). Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, phospho-AKT and CDK4 are oncogenic signaling molecules critically involved in cell growth, differentiation and apoptosis, processes which are important in cancer cells. The degradation of one or multiple oncoproteins is believed to produce the anti-tumor effects observed with Hsp90 inhibitors.

The Hsp90 family of chaperones is comprised of four members: Hsp90$\alpha$ and Hsp90$\beta$ both located in the cytosol, GRP94 in the endoplasmic reticulum, and TRAP1 in the mitochondria (Csermely et al., 1998). Hsp90 is the most abundant cellular chaperone, constituting about 1% - 2% of total protein (Jakob and Buchner, 1994).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, in vivo, Hsp90 proteins work as subunits of large, dynamic protein complexes. Hsp90 is essential for eukaryotic cell survival and is overexpressed in many tumors.

BRIEF SUMMARY OF THE INVENTION

It has now been found that a combination comprising at least one Hsp90 inhibitor compound and at least one HER2 inhibitor, e.g. as defined below, has a beneficial effect on proliferative disorders, including without limitation, e.g. solid tumors, e.g. breast cancer.

The present invention provides a pharmaceutical combination comprising (a) a HSP90 inhibitor and (b) a HER2 inhibitor, wherein the HSP90 inhibitor is 5-(2,4-Dihydroxy-5-isopropyl-phenyl)-4-(4-morpholin-4- ylmethyl-phenyl)-isoxazole-3-carboxylic acid ethylamide, or a pharmaceutically acceptable salt thereof; and the HER2 inhibitor is trastuzumab.

There is also provided the above combination as a kit.

There is also provided a combination as described above in the form of a pharmaceutical composition.

The present invention provides a combination comprising at least one HSP90 inhibitor compound and at least one HER2 inhibitor for use in a method of treating a proliferative disease comprising administering to a patient in need thereof an effective amount of said HSP90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, as a first pharmaceutical agent, and said HER2 inhibitor which is trastuzumab as a second pharmaceutical agent.

There is also provided a combination for use as described above wherein the proliferative disorder is breast cancer.

The present invention also provides the above combination for use wherein each agent is administered simultaneously or sequentially and in any order.

The present invention also provides the above combination for use wherein each agent is administered separately or as a fixed combination.

BRIEF DESCRIPTION OF THE DRAWINGS

Fig. 1 shows the effect of compound I (5-(2,4-Dihydroxy-5-isopropyl-phenyl)-4-(4-morpholin-4-ylmethyl-phenyl)-isoxazole-3-carboxylic acid ethylamide also known as AUY922) with trastuzumab in the BT-474 breast cancer xenograft model.

DETAILED DESCRIPTION OF THE INVENTION

" As used herein, the term "pharmaceutically acceptable salts" refers to the nontoxic acid or alkaline earth metal salts of compounds of the invention. These salts can be prepared in situ during the final isolation and purification of the compounds, or by separately reacting the base or acid functions with a suitable organic or inorganic acid or base, respectively. Representative salts include, but are not limited to, the following: acetate, adipate, alginate, citrate, aspartate, benzoate, benzenesulfonate, bisulfate, butyrate, camphorate, camphorsulfonate, digluconate, cyclopentanepropionate, dodecylsulfate, ethanesulfonate, glucoheptanoate, glycerophosphate, hemi-sulfate, heptanoate, hexanoate, fumarate, hydrochloride, hydrobromide, hydroiodide, 2-hydroxyethanesulfonate, lactate, maleate, methanesulfonate, nicotinate, 2-napthalenesulfonate, oxalate, pamoate, pectinate, persulfate, 3-phenylproionate, picrate, pivalate, propionate, succinate, sulfate, tartrate, thiocyanate, p-toluenesulfonate, and undecanoate. Also, the basic nitrogen-containing groups can be quaternized with such agents as alkyl halides, such as methyl, ethyl, propyl, and butyl chloride, bromides, and iodides; dialkyl sulfates like dimethyl, diethyl, dibutyl, and diamyl sulfates, long chain halides such as decyl, lauryl, myristyl, and stearyl chlorides, bromides and iodides, aralkyl halides like benzyl and phenethyl bromides, and others. Water or oil-soluble or dispersible products are thereby obtained.

Examples of acids that may be employed to form pharmaceutically acceptable acid addition salts include such inorganic acids as hydrochloric acid, sulfuric acid and phosphoric acid and such organic acids as oxalic acid, maleic acid, methanesulfonic acid, succinic acid and citric acid. Basic addition salts can be prepared in situ during the final isolation and purification of the compound, or separately by reacting carboxylic acid moieties with a suitable base such as the hydroxide, carbonate or bicarbonate of a pharmaceutically acceptable metal cation or with ammonia, or an organic primary, secondary or tertiary amine. Pharmaceutically acceptable salts include, but are not limited to, cations based on the alkali and alkaline earth metals, such as sodium, lithium, potassium, calcium, magnesium, aluminum salts and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. Other representative organic amines useful for the formation of base addition salts include diethylamine, ethylenediamine, ethanolamine, diethanolamine, piperazine, and the like.

The term "pharmaceutically acceptable prodrugs" as used herein refers to those prodrugs of the compounds of the present invention which are, within the scope of sound medical judgment, suitable for use in contact with the tissues of humans and lower animals without undue toxicity, irritation, allergic response, and the like, commensurate with a reasonable benefit/risk ratio, and effective for their intended use, as well as the zwitterionic forms, where possible, of the compounds of the invention. The term "prodrug" refers to compounds that are rapidly transformed in vivo to yield the parent compound of the above formula, for example by hydrolysis in blood, such as an ester prodrug. A thorough discussion is provided in Higuchi, T., and V. Stella, "Pro-drugs as Novel Delivery Systems," A.C.S. Symposium Series 14, and in "Bioreversible Carriers in Drug Design," in Edward B. Roche (ed.), American Pharmaceutical Association, Pergamon Press, 1987, both of which are incorporated herein by reference.

In embodiments, a pharmaceutical composition according to the invention comprises a first pharmaceutical component and a second pharmaceutical component in a pharmaceutically acceptable carrier.

The present disclosure describes the use of compounds consisting of those of formula (E), and regioisomers thereof, and their salts, solvates and hydrates, and prodrugs thereof:

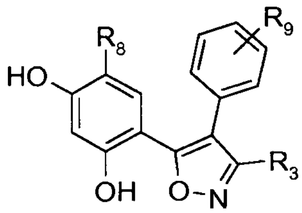

(E)

wherein $R_3$ represents a carboxamide group (such as ethylaminocarbonyl $CH_3CH_2NHC(=O)$-, or isopropylaminocarbonyl $(CH_3)_2CHNHC(=O)$-); $R_9$ represents-$CH_2NR^{10}R^{11}$ or -$NR^{10}R^{11}$ wherein the substituted amino group -$NR^{10}R^{11}$ is a solubilising group, (such as morpholinyl, piperidinyl, piperazinyl, pyrrolidinyl, ethylamino, isopropylamino, diethylamino, cyclohexylamino, cyclopentylamino, methoxyethylamino, piperidin-4-yl, N-acetylpiperazinyl, N-methylpiperazinyl, methylsulfonylamino, thiomorpholinyl, thiomorpholinyl-dioxide, 4-hydroxyethylpiperidinyl, and 4-hydroxypiperidinyl); and $R_8$ represents an optional substituent, especially a small lipophilic group (such as ethyl, isopropyl, bromo, or chloro).

In such 5-substituted, 2,4-diyhdroxy phenyl compounds of the invention, the hydroxyl groups may be protected by groups which are cleaved in the body to release the hydroxyl groups. Known prodrug-type groups of this kind which are cleaved to hydroxyls include alkylcarbonyloxy groups such as methylcarbonyloxy, and alkylaminocarbonyloxy groups such as dialkylamino- or isopropylamino-carbonyloxy.

A specific compound with which the disclosure is concerned include particularly the following, and its salts, N-oxides, hydrates and solvates, and prodrugs thereof:

5-(2,4-Dihydroxy-5-isopropyl-phenyl)-4-(4-morpholin-4-ylmethyl-phenyl)-isoxazole-3-carboxylic acid ethylamide

Compounds within the scope of formula (E) and the process for their manufacture are disclosed in WO 04/072051 published on August 26, 2004. A preferred compound is 5-(2,4-Dihydroxy-5-isopropyl-phenyl)-4-(4-morpholin-4-ylmethyl-phenyl)-isoxazole-3-carboxylic acid ethylamide.

The present disclosure relates to a pharmaceutical combination comprising

a) a compound of formula (E)

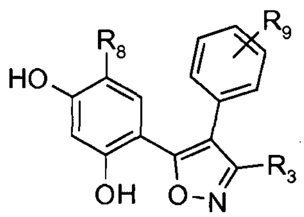

(E)

wherein $R_3$ is selected from ethylaminocarbonyl $CH_3CH_2NHC(=O)$- or isopropylaminocarbonyl $(CH_3)_2CHNHC(=O)$-),
$R_8$ is selected from ethyl, isopropyl, bromo, or chloro; and
$R_9$ is selected from morpholinyl, piperidinyl, piperazinyl, pyrrolidinyl, ethylamino, isopropylamino, diethylamino, cyclohexylamino, cyclopentylamino, methoxyethylamino, piperidin-4-yl, N-acetylpiperazinyl, N-methylpiperazinyl, methylsulfonylamino, thiomorpholinyl, thiomorpholinyl-dioxide, 4-hydroxyethylpiperidinyl or 4-hydroxypiperidinyl; and

b) at least one HER2 (ErbB2) inhibitor

The compound of formula (E) may be a Hsp90 inhibitor.

A preferred compound of formula (E) is 5-(2,4-Dihydroxy-5-isopropyl-phenyl)-4-(4-morpholin-4-ylmethyl-phenyl)-isoxazole-3-carboxylic acid ethylamide.

In one aspect the present invention provides the use of 5-(2,4-Dihydroxy-5-isopropyl-phenyl)-4-(4-morpholin-4-ylmethyl-phenyl)-isoxazole-3-carboxylic acid ethylamide and at least one HER2 (ErbB2) inhibitor for the manufacture of a medicament for the treatment or prevention of a proliferative disease, wherein said HER2 inhibitor is trastuzumab.

In a further aspect the present invention provides 5-(2,4-Dihydroxy-5-isopropylphenyl)-4-(4-morpholin-4-ylmethyl-phenyl)-isoxazole-3-carboxylic acid ethylamide and at least one HER2 (ErbB2) inhibitor for use in treating or preventing a proliferative disease, wherein said HER2 inhibitor is trastuzumab.

The present disclosure further relates to combinations comprising compounds targeting, decreasing or inhibiting the activity of the epidermal growth factor (EGFR) family of receptor tyrosine kinases (ErbB1 (EGFR1), ErbB2 (EGFR2, HER2), ErbB3 (EGFR3), ErbB4 (EGFR4) as homo- or heterodimers), such as compounds which target, decrease or inhibit the activity of the epidermal growth factor receptor family are especially compounds, proteins or antibodies which inhibit members of the EGF receptor tyrosine kinase family, e.g., EGF receptor, ErbB1, ErbB2, ErbB3 and ErbB4 or bind to EGF or EGF-related ligands, and are in particular those compounds, proteins or monoclonal antibodies generically and specifically disclosed in US 5,677,171, which is hereby incorporated into the present application by reference. Comprised are likewise the corresponding stereoisomers as well as the corresponding crystal modifications, e.g. solvates

and polymorphs, which are disclosed therein. More preferred is trastuzumab also marketed under the tradename Herceptin™. Trastuzumab, a monoclonal antibody, works by interfering with one of the ways in which breast cancer cells grow and divide. Some breast cancer cells overexpress a protein known as HER2 (ErbB2) on their cell surface. Trastuzumab blocks the positive growth signal HER2 overexpression supplies to the tumor by attaching itself to the HER2 (ErbB2) protein and acting as a HER2 (ErbB2) inhibitor. This inhibits tumor cell division and growth. Trastuzumab also works by attracting the body's own immune cells to help destroy the cancer cells. Trastuzumab is approved for the treatment of breast cancer whose tumors overexpress the HER2 (ErbB2) protein. Another example of a HER2 (ErbB2) inhibitor is lapatinib. An example of a HER1 (ErbB1) inhibitor is erlotinib.

In each case where citations of patent applications are given above, the pharmaceutically acceptable salts, the corresponding racemates, diastereoisomers, enantiomers, tautomers, as well as the corresponding crystal modifications of above disclosed compounds where present, e.g. solvates, hydrates and polymorphs, are also disclosed therein. The compounds used as active ingredients in the combinations of the invention can be prepared and administered as described in the cited documents, respectively. Also within the scope of this invention is the combination of more than two separate active ingredients as set forth above, i.e., a pharmaceutical combination within the scope of this invention could include three active ingredients or more.

Suitable clinical studies may be, for example, open label, dose escalation studies in patients with proliferative diseases. Such studies prove in particular the synergism of the active ingredients of the combination of the invention. The beneficial effects on proliferative diseases may be determined directly through the results of these studies which are known as such to a person skilled in the art. Such studies may be, in particular, suitable to compare the effects of a monotherapy using the active ingredients and a combination of the invention. Preferably, the dose of agent (a) is escalated until the Maximum Tolerated Dosage is reached, and agent (b) is administered with a fixed dose. Alternatively, the agent (a) may be administered in a fixed dose and the dose of agent (b) may be escalated. Each patient may receive doses of the agent (a) either daily or intermittent. The efficacy of the treatment may be determined in such studies, e.g., after 12, 18 or 24 weeks by evaluation of symptom scores every 6 weeks.

The administration of a pharmaceutical combination of the invention may result not only in a beneficial effect, e.g. a synergistic therapeutic effect, e.g. with regard to alleviating, delaying progression of or inhibiting the symptoms, but also in further surprising beneficial effects, e.g. fewer side-effects, an improved quality of life or a decreased morbidity, compared with a monotherapy applying only one of the pharmaceutically active ingredients used in the combination of the invention.

A further benefit may be that lower doses of the active ingredients of the combination of the invention may be used, for example, that the dosages need not only often be smaller but may also be applied less frequently, which may diminish the incidence or severity of side-effects. This is in accordance with the desires and requirements of the patients to be treated.

It is one objective of this invention to provide a pharmaceutical composition comprising a quantity of a first component and a second component as previously described, which may be jointly therapeutically effective at targeting or preventing proliferative diseases. These first and second components may be provided for administration in a fixed combination, i.e. in a single galenical composition, which may be prepared in a manner known per se, suitable for enteral, such as oral or rectal, and parenteral administration to mammals (warm-blooded animals), including humans in combination with one or more pharmaceutically acceptable carriers or diluents, especially suitable for enteral or parenteral application.

Alternatively, the first component and the second component may be provided as separate pharmaceutical compositions in a kit.

The pharmaceutical compositions for separate administration of the first component and the second component may be prepared in a manner known per se and are those suitable for enteral, such as oral or rectal, and parenteral administration to mammals (warm-blooded animals), including humans. Each such composition for separate administration comprises a therapeutically effective amount of at least one pharmacologically active component in combination with one or more pharmaceutically acceptable carriers or diluents.

Suitable pharmaceutical compositions may contain, for example, from about 0.1 % to about 99.9%, preferably from about 1 % to about 60 %, of the active ingredient(s). Pharmaceutical preparations for the combination therapy for enteral or parenteral administration are, for example, those in unit dosage forms, such as sugar-coated tablets, tablets, capsules or suppositories, or ampoules. If not indicated otherwise, these are prepared in a manner known per se, for example by means of conventional mixing, granulating, sugar-coating, dissolving or lyophilizing processes. It will be appreciated that the unit content of a pharmaceutical component contained in an individual dose of each dosage form need not in itself constitute an effective amount since the necessary effective amount may be reached by administration of a plurality of dosage units.

In a method of treating proliferative disease, the first component and the second component may be administered together, sequentially or separately. The first and second components may be delivered in one combined unit dosage form or in multiple separate unit dosage forms.

In particular, a therapeutically effective amount of each of the pharmaceutical components of the invention may be administered simultaneously or sequentially and in any order, and the components may be administered separately or as a fixed combination. For example, here is described a method of preventing or treating proliferative diseases which may comprise (i) administration of the first component in free or pharmaceutically acceptable salt form and (ii) administration of the second component in free or pharmaceutically acceptable salt form, simultaneously or sequentially in any order, in jointly therapeutically effective amounts, preferably in synergistically effective amounts, e.g. in daily or intermittently dosages corresponding to the amounts described herein. The individual combination components of the combination of the invention may be administered separately at different times during the course of therapy or concurrently in divided or single combination forms. Furthermore, the term administering also encompasses the use of a prodrug of a combination component that convert in vivo to the combination partner as such. The instant invention is therefore to be understood as embracing all such regimens of simultaneous or alternating treatment and the term "administering" is to be interpreted accordingly.

The effective dosage of each of the components employed in the combination of the invention may vary depending on the particular compound or pharmaceutical composition employed, the mode of administration, the condition being treated, the severity of the condition being treated. Thus, the dosage regimen of the combination of the invention is selected in accordance with a variety of factors including the route of administration and the renal and hepatic function of the patient. A clinician or physician of ordinary skill can readily determine and prescribe the effective amount of the single active ingredients required to alleviate, counter or arrest the progress of the condition. Optimal precision in achieving concentration of the active ingredients within the range that yields efficacy without toxicity requires a regimen based on the kinetics of the active ingredients' availability to target sites.

The amount of active ingredient that may be combined with carrier materials to produce a single dosage form will vary depending upon the host treated and the particular mode of administration. It will be understood, however, that the specific dose level for any particular patient will depend upon a variety of factors including the activity of the specific compound employed, the age, body weight, general health, sex, diet, time of administration, route of administration, rate of excretion, drug combination, and the severity of the particular disease undergoing therapy. The therapeutically effective amount for a given situation can be readily determined by routine experimentation and is within the skill and judgment of the ordinary clinician.

For purposes of the present invention, a therapeutically effective dose will generally be a total daily dose administered to a host in single or divided doses may be in amounts, for example, of from 0.001 to 1000 mg/kg body weight daily and more preferred from 1.0 to 30 mg/kg body weight daily. Dosage unit compositions may contain such amounts of submultiples thereof to make up the daily dose.

The compounds according to Formula (I), the HER2 inhibitors and the pharmaceutical compositions comprising these active ingredients, may be administered orally, parenterally, sublingually, by aerosolization or inhalation spray, rectally, or topically in dosage unit formulations containing conventional nontoxic pharmaceutically acceptable carriers, adjuvants, and vehicles as desired. Topical administration may also involve the use of transdermal administration such as transdermal patches or ionophoresis devices. The term parenteral as used herein includes subcutaneous injections, intravenous, intramuscular, intrasternal injection, or infusion techniques.

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in 1,3-propanediol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono- or di-glycerides. In addition, fatty acids such as oleic acid find use in the preparation of injectables.

Suppositories for rectal administration of the drug can be prepared by mixing the drug with a suitable nonirritating excipient such as cocoa butter and polyethylene glycols, which are solid at ordinary temperatures but liquid at the rectal temperature and will therefore melt in the rectum and release the drug.

Solid dosage forms for oral administration may include capsules, tablets, pills, powders, and granules. In such solid dosage forms, the active compound may be admixed with at least one inert diluent such as sucrose lactose or starch. Such dosage forms may also comprise, as is normal practice, additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

The compounds according to Formula (I), HER2 inhibitors and pharmaceutical compositions described herein can also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes can be used. The present compositions in liposome form can contain, in addition to a compound of the present invention, stabilizers, preservatives, excipients, and the like. The preferred lipids are the phospholipids and phosphatidyl cholines (lecithins), both natural and synthetic. Methods to form liposomes are known in the art. See, for example, Prescott (ed.), "Methods in Cell Biology," Volume XIV, Academic Press, New York, 1976, p. 33 et seq.

The terms "proliferative disease" and "proliferative disorder" include but are not restricted to cancer, e.g. solid tumors, e.g. breast cancer.

EXAMPLES

Example 1: Antitumor effect of compound I 5-(2,4-Dihydroxy-5-isopropyl-phenyl)-4-(4-morpholin-4-ylmethyl-phenyl)-isoxazole-3-carboxylic acid ethylamide when used in combination with trastuzumab in a human breast carcinoma xenograft model in nude mice

The estrogen receptor positive cell line BT-474 (ATCC, number HTB-20) which is a human breast carcinoma cell line is used. The cells are grown in DMEM high glucose (4.5 g/l) supplemented with 10% FCS, 200 mM L-glutamine and 1% sodium pyruvate.

In preparation for cell inoculation, each mouse is subcutaneously implanted on the upper dorsal side with a 17β-Estradio pellet (25 microgram/day; 90 day release) using a trocar needle. BT-474 cells (5x10^6) are injected in 200 microliter Matrigel:HBSS (1:1 vol) (BD Matrigel™ Basement Membrane Matrix). The injection site is subcutaneously in the right flank. Treatment with compound I is initiated when the average tumor volume reached approximately 100 $mm^3$. Tumor growth is monitored at regular intervals. The xenograft tumor sizes is measured manually with calipers and the tumor volume is estimated using the formula: ($W \times L^2 \times \pi/6$), where width (W) and length (L) are the two largest diameters. Results are presented as mean ± SEM. Tumor data are analyzed by ANOVA with post hoc Dunnet's test for comparison of treatment versus control group.

As a measure of efficacy the %T/C value is calculated at the end of the experiment according to:

$$(\Delta \text{tumor volume}_{\text{treated}}/\Delta \text{tumor volume}_{\text{control}})*100$$

Where Δtumor volumes represent the mean tumor volume on the evaluation day minus the mean tumor volume at the start of the experiment.

Tumor regression is presented as the relative absolute change in tumor volume from the start to the end of the experiment (-(Tumor $\text{volume}_{\text{evaluation day}}$ - Tumor $\text{volume}_{\text{start day}}$)/ Tumor $\text{volume}_{\text{start day}}$* 100).

The antitumor effect of compound I and trastazumab is evaluated in the BT-474 xenograft model (Fig. 1). The treatment period is 21 days and each treatment group consists of 8 tumor bearing animals. The tumor sizes in the treatment groups are compared to those of the vehicle treated groups and the effect is expressed as %T/C or regression when applicable. Trastuzumab is administered intravenously (i.v.) twice per week at a dose level of 5 mg/kg. Compound I is administered i.v. once per week at a dose of 15 mg/kg.

Table 1 shows that 5 mg/kg trastuzumab which is administered i.v. twice per week results in 57% reduction of tumor burden. Compound I which is administered once per week (qw) at a dose of 15 mg/kg gives a 4% reduction in tumor burden (no statistically significant difference from vehicle treated animals). The combination of the two agents results in a strongly enhanced antitumor effect compared with either one alone as 22% tumor regression was observed. These data provide strong pre-clinical support for combining trastuzumab with compound I against ERBB2 positive breast cancer.

Table 1

| Compound | Dose, schedule, route | T/C (%) | Regresssion (%) | ΔTumor volume ($mm^3$) |
|---|---|---|---|---|
| Vehicle control | 10 ml/kg, qw, i.v. | 100 | - | 970 ± 208 |
| Trastuzumab | 5 mg/kg, 2qw, i.v. | 43 | - | 415 ±155 |
| Compound I | 15 mg/kg, qw, i.v. | 96 | - | 929 ± 152 |

(continued)

| Compound | Dose, schedule, route | T/C (%) | Regresssion (%) | ΔTumor volume ($mm^3$) |
|---|---|---|---|---|
| Compound I + Trastuzumab | 15 mg/kg, qw, i.v.<br>5 mg/kg, 2qw, i.v. | - | 22 | -48 ± 31* |
| * P < 0.05; one-way ANOVA post hoc Dunnet's test. | | | | |

## Claims

1. A pharmaceutical combination comprising

(a) a HSP90 inhibitor and
(b) a HER2 inhibitor,

wherein the HSP90 inhibitor is 5-(2,4-Dihydroxy-5-isopropyl-phenyl)-4-(4-morpholin-4-ylmethyl-phenyl)-isoxazole-3-carboxylic acid ethylamide,
or a pharmaceutically acceptable salt thereof;
and the HER2 inhibitor is trastuzumab.

2. The combination of claim 1, wherein the combination is a kit.

3. The combination of claim 1, wherein the combination is a pharmaceutical composition.

4. A combination comprising at least one HSP90 inhibitor compound and at least one HER2 inhibitor for use in a method of treating a proliferative disease comprising administering to a patient in need thereof an effective amount of said HSP90 inhibitor which is 5-(2,4-dihydroxy-5-isopropyl-phenyl)-4-(4-morpholin-4-ylmethyl-phenyl)-isoxazole-3-carboxylic acid ethylamide, or a pharmaceutically acceptable salt thereof, as a first pharmaceutical agent, and said HER2 inhibitor which is trastuzumab as a second pharmaceutical agent.

5. The combination for use according to claim 4, wherein said proliferative disorder is breast cancer.

6. The combination for use according to claim 4, wherein each agent is administered simultaneously or sequentially and in any order.

7. The combination for use according to claim 4, wherein each agent is administered separately or as a fixed combination. 1

## Patentansprüche

1. Arzneimittelkombination enthaltend

(a) einen HSP90-Hemmer und
(b) einen HER2-Hemmer,

wobei der HSP90-Hemmer 5-(2,4-Dihydroxy-5-isopropyl-phenyl)-4-(4-morpholin-4-ylmethyl-phenyl)-isoxazol-3-carbonsäure-ethylamid ist,
oder ein pharmazeutisch akzeptables Salz desselben;
und der HER2-Hemmer Trastuzumab ist.

2. Kombination nach Anspruch 1, wobei die Kombination ein Kit ist.

3. Kombination nach Anspruch 1, wobei die Kombination eine pharmazeutische Zusammensetzung ist.

4. Kombination enthaltend mindestens eine HSP90-Hemmer-Verbindung und mindestens einen HER2-Hemmer für

deren Verwendung in einem Verfahren zur Behandlung von einer proliferativen Erkrankung, umfassend das Verabreichen einer wirksamen Menge von
dem genannten HSP90-Hemmer, welcher 5-(2,4-Dihydroxy-5-isopropyl-phenyl)-4-(4-morpholin-4-ylmethyl-phenyl)-isoxazol-3-carbonsäure-ethylamid ist, oder ein pharmazeutisch akzeptables Salz desselben, als erstes pharmazeutisches Mittel, und
dem genannten HER2-Hemmer, welcher Trastuzumab ist, als zweites pharmazeutisches Mittel,
an einen bedürftigen Patienten.

**5.** Kombination für deren Verwendung nach Anspruch 4, wobei die genannte proliferative Erkrankung Brustkrebs ist.

**6.** Kombination für deren Verwendung nach Anspruch 4, wobei jedes Mittel gleichzeitig oder sequenziell und in einer beliebigen Reihenfolge verabreicht wird.

**7.** Kombination für deren Verwendung nach Anspruch 4, wobei jedes Mittel separat oder als festgelegte Kombination verabreicht wird.

## Revendications

**1.** Combinaison pharmaceutique comprenant

(a) un inhibiteur de HSP90 et
(b) un inhibiteur de HER2,

dans laquelle l'inhibiteur de HSP90 est de l'éthylamide de l'acide 5-(2,4-dihydroxy-5-isopropyl-phényl)-4-(4-morpholin-4-ylméthyl-phényl)-isoxazole-3-carboxylique, ou un des ses sels pharmaceutiquement acceptables;
et l'inhibiteur de HER2 est du trastuzumab.

**2.** Combinaison selon la revendication 1, dans laquelle la combinaison est un kit.

**3.** Combinaison selon la revendication 1, dans laquelle la combinaison est une composition pharmaceutique.

**4.** Combinaison comprenant au moins un composé inhibiteur de HSP90 et au moins un inhibiteur de HER2 pour être utilisée dans un procédé de traitement d'une maladie proliférative comprenant l'administration à un patient en ayant besoin d'une quantité effective dudit inhibiteur de HSP90 qui est de l'éthylamide de l'acide 5-(2,4-dihydroxy-5-isopropyl-phényl)-4-(4-morpholin-4-ylméthyl-phényl)-isoxazole-3-carboxylique, ou un des ses sels pharmaceutiquement acceptables, comme premier agent pharmaceutique, et
ledit inhibiteur de HER2 qui est du trastuzumab comme deuxième agent pharmaceutique.

**5.** Combinaison pour être utilisée selon la revendication 4, dans laquelle ledit trouble prolifératif est le cancer du sein.

**6.** Combinaison pour être utilisée selon la revendication 4, dans laquelle chaque agent est administré simultanément ou séquentiellement et dans n'importe quel ordre.

**7.** Combinaison pour être utilisée selon la revendication 4, dans laquelle chaque agent est administré séparément ou comme une combinaison fixe.

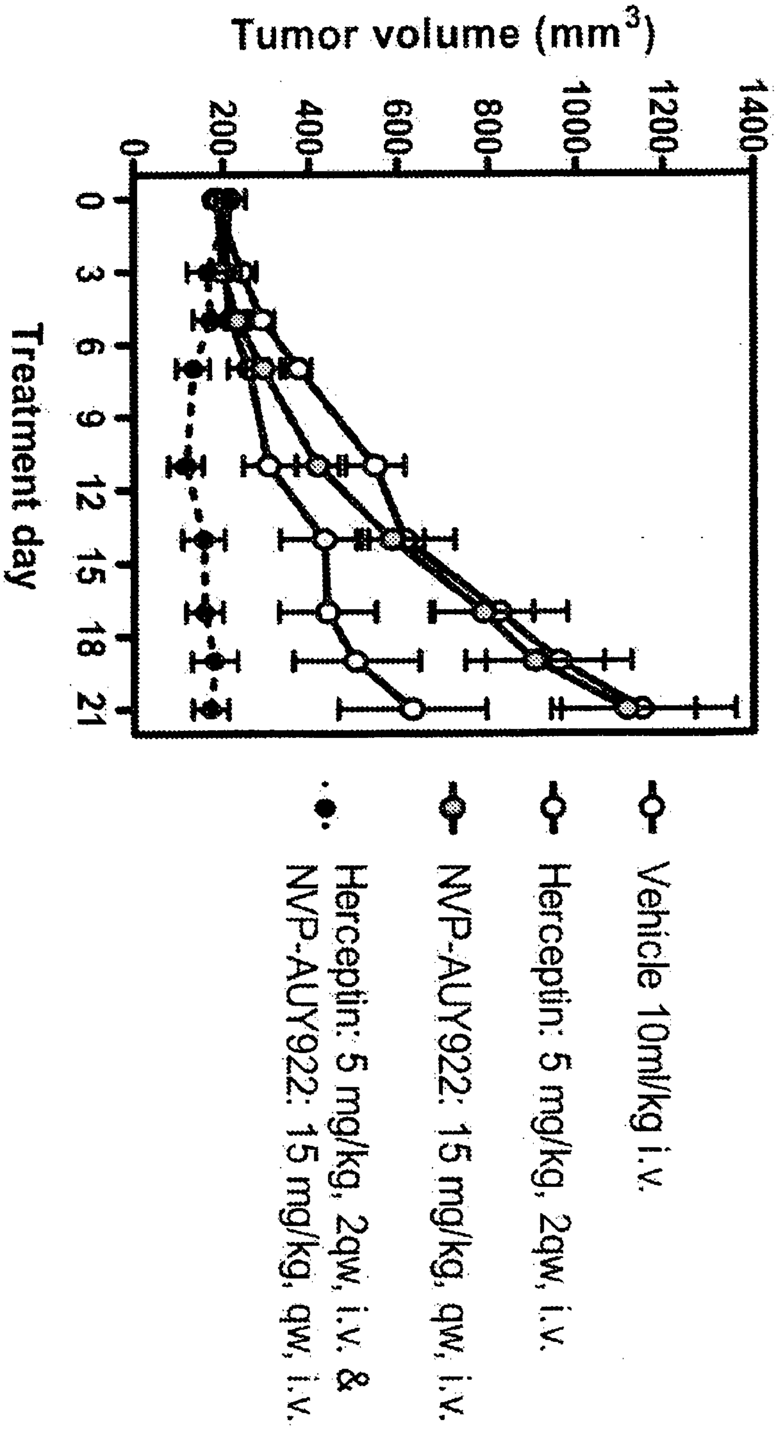
Tumor volume (mm3)
0
200
400
600
800
1000
1200
1400
0
3
6
9
12
15
18
21
Treatment day
Vehicle 10ml/kg i.v.
Herceptin: 5 mg/kg, 2qw, i.v.
NVP-AUY922: 15 mg/kg, qw, i.v.
Herceptin: 5 mg/kg, 2qw, i.v. &
NVP-AUY922: 15 mg/kg, qw, i.v.

FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 08170255 A
- EP 08170261 A
- WO 04072051 A
- US 5677171 A

### Non-patent literature cited in the description

- Pro-drugs as Novel Delivery Systems. **HIGUCHI, T. ; V. STELLA.** A.C.S. Symposium Series. 14
- Bioreversible Carriers in Drug Design. American Pharmaceutical Association, Pergamon Press, 1987
- Methods in Cell Biology. Academic Press, 1976, vol. XIV, 33